# EUROPEAN PATENT APPLICATION

(11) **EP 4 596 714 A1**
(43) Date of publication of application: **06.08.2025**
(21) Application number: 24155690.1
(22) Date of filing: 05.02.2024
(51) Int. Cl.: C12Q 1/6816, G01N 33/532

(54) **MARKER FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect a marker (100, 400, 500, 600) for analysing a biological sample is provided. The marker (100, 400, 500, 600) comprises an affinity reagent (102), in particular configured to attach specifically to a target analyte of the biological sample. The marker (100, 400, 500, 600) further comprises at least one labelling moiety (104, 602, 604), and at least one linker oligonucleotide (106, 204, 404, 506a, 506b, 506c). The linker oligonucleotide (106, 204, 404, 506a, 506b, 506c) is attached to the affinity reagent (102) and the at least one labelling moiety (104, 602, 604) is attached to the linker oligonucleotide (106, 204, 404, 506a, 506b, 506c). In a further aspect, a method for generating the marker (100, 400, 500, 600) is provided.

## Description

### Technical field

The invention relates to a marker for analysing a biological sample and a method for generating the marker.

### Background

Labels and markers are frequently used when analysing biological samples, for example in fluorescent microscopy. Markers for fluorescent microscopy generally comprise affinity reagents such as antibodies and labels attached to the affinity reagents in order to enable specifically attaching the labels to a target analyte in a biological sample. This allows the identification and/or localisation of the target analyte in the biological sample. The ubiquitous use of these markers and labels, for example in cyclical staining methods, results in a drive to improve the efficiency of producing these markers and labels, as well as their robustness in use.

A particular issue with labels and markers known in the art is that each part of the label or marker is often generated individually. When assembling markers, the individual parts need to be assembled resulting in a complex production. These steps are generally associated with losses due to limited efficiency of the reactions and subsequent purification losses. Further, the attachment of the individual parts to each other is often reversible, for example, attachments via hybridisation. These attachments result in less robust markers and a shorter shelf life of these markers.

### Summary

It is an object to provide labels for analysing a biological sample that are easy to assemble and robust as well as an efficient method to generate the labels.

The aforementioned object is achieved by the subject-matter of the independent claims. Advantageous embodiments are defined in the dependent claims and the following description.

In a first aspect a marker for analysing a biological sample is provided. The marker comprises an affinity reagent, in particular configured to attach specifically to a target analyte of the biological sample. The marker further comprises at least one labelling moiety, and at least one linker oligonucleotide. The linker oligonucleotide is, preferably directly, attached to the affinity reagent and the at least one labelling moiety is attached to the linker oligonucleotide, in particular in order to attach the labelling moiety to the affinity reagent.

The at least one labelling moiety is preferably optically detectable. For example, the labelling moiety may be a fluorophore, a fluorescent dye, a fluorescent protein, a fluorescent nanostructure, a polymer dye, a quantum dot, a polymer dot, an enzyme (e.g. horse raddish peroxidase, alkaline phosphatase), an oligonucleotide sequence, a metal tag, a radioactive label, a Raman label. Alternatively, the labelling moiety may be a polyyne. A labelling moiety may comprise a plurality of the aforementioned molecules or structures. In some cases, the labelling moiety may be configured to form a signal-generating molecule or structure. For example, the labelling moiety may be fluorogenic molecule configured to react with another molecule or under a particular condition to become fluorescent, as for instance described in the European patent application with the application number 24152941.1, or as for instance described in the PCT application with the application number PCT/EP2023/081541, the complete content of these two applications is incorporated herein by reference. In a further example the fluorescent structure may be an aptamer beacon, or an aptamer configured to bind a fluorescent dye or other reporter molecule configured to generate a detectable signal, as for instance described in the European patent application with the application number 23209753.5, the complete content is incorporated herein by reference.

In a preferred embodiment, the linker oligonucleotide is a single strand nucleic acid molecule. This enables generating the marker efficiently. The linker oligonucleotide may be a DNA and/or a nucleic acid analogue (XNA), for example. The linker oligonucleotide may comprise PT-DNA, LNA, PNA, or L-DNA, which are resistant to nucleases. Such resistance may be desired in certain applications. In some cases, it may be desirable to use nuclease-sensitive linker oligonucleotides. The linker oligonucleotide may also comprise non-naturally occurring nucleobases or backbones.

The length of the linker oligonucleotide is preferably between 4 to 120 nucleotides, more preferably between 15 to 60 nucleotides.

Preferably, the marker comprises a plurality of labelling moieties, each one of the labelling moieties is attached to the affinity reagent by one linker oligonucleotide. This enables generating a bright label or marker that is also compact. The attachment of or attaching the linker oligonucleotide to the affinity reagent may be site specific, in particular.

Preferably, a plurality of labelling moieties is attached to the at least one linker oligonucleotide. This enables generating a bright label or marker that is also compact.

In a preferred embodiment, the affinity reagent is a polymer. For example, the affinity reagent may comprise amino acids or nucleic acids. In particular, the affinity reagent may be a protein, an antibody, an antibody fragment, a nanobody, a polymeric binder or an aptamer. This enables a marker with a high specificity of binding to a particular target analyte. The antibody fragment may be a Fab or a nanobody. Alternatively, the affinity reagent may be an aptamer.

Preferably, the attachment of the linker oligonucleotide to the affinity reagent is at least one covalent bond, and the attachment of the at least one labelling moiety to the linker oligonucleotide is at least one covalent bond. This enables a robust marker. In particular, the linker oligonucleotide is not hybridised to the affinity reagent and/or to the at least one labelling moiety. Thus and in particular, the attachment of the linker oligonucleotide to the affinity reagent and/or to the at least one labelling moiety is not based on hybridisation of complementary nucleic acids. In particular, the labelling moiety is attached to the affinity reagent via the linker oligonucleotide and via covalent bonds.

In a further aspect, a method for generating a marker, in particular according to an embodiment discussed above or in the description of this document, is provided. The method is for analysing a biological sample and comprises the following steps: providing an affinity reagent and at least one linker oligonucleotide with at least one labelling moiety attached to the at least one linker oligonucleotide and attaching the at least one linker oligonucleotide to the affinity reagent. In particular, this may form or generate the marker. The method enables efficiently generating a robust marker. In particular, the labelling moiety may be attached to a first end of the linker oligonucleotide, a second end of the linker oligonucleotide may then be attached to the affinity reagent. In particular, the step of attaching the at least one linker oligonucleotide to the affinity reagent comprises forming a covalent bond between the linker oligonucleotide and the affinity reagent.

A number of chemistries suited to perform such covalent coupling of oligonucleotides to proteins, such as antibodies, include N-Hydroxysuccinimide (NHS coupling chemistry), homobifunctional linker disuccinimidyl suberate (DSS) coupling chemistry [Li G, Moellering RE. A Concise, Modular Antibody-Oligonucleotide Conjugation Strategy Based on Disuccinimidyl Ester Activation Chemistry. Chembiochem. 2019 Jun 14;20(12):1599-1605. doi: 10.1002/cbic.201900027. Epub 2019 May 3. PMID: 30767357; PMCID: PMC6773266.], site-specific click chemistry, dibenzocyclooctyne (DBCO)/azide click chemistry, maleimide coupling chemistry.

Similarly, the labelling moiety may be attached to the linker oligonucleotide by a covalent bond, for example.

The method may be carried out to generate a plurality of markers, to that end a plurality of affinity reagents and linker oligonucleotides with labelling moieties may be provided.

In particular, the method is suited for bench-scale conjugation of antibodies to linker oligonucleotides by the user as well as for production-scale conjugation. When performed by the user for screening applications it may be desirable to perform the conjugation in small volumes and to dispense the components using acoustic liquid dispensing systems like for example the Echo from Beckman-Coulter.

Preferably, the step of attaching the at least one linker oligonucleotide comprises attaching at least one attachment oligonucleotide to the affinity reagent and further comprises ligating the at least one linker oligonucleotide, for example its 5' end, to the at least one attachment oligonucleotide, for example its 3' end. The ligation may be performed using T4 DNA ligase for example, which is ATP-dependent and requires Mg2+ as a cofactor. The ligation step therefore is carried out using a suitable buffer comprising these components preferably at RT. A standard T4 ligase buffer may be used or a modification thereof. Other DNA ligases may be used for the ligation, for example: E. coli DNA Ligase, Taq DNA ligase, Pfu DNA ligase, thermostable DNA ligases. Such DNA ligases catalyse the formation of a covalent phosphodiester bond between the 3' hydroxyl group of an acceptor nucleotide and the 5' phosphate group of a donor nucleotide [source: https://en.wikipedia.org/wiki/DNA_ligase]. For this reason, the linker and attachment oligonucleotides need to comprise the end groups for an enzymatic ligation to work.

This enables an efficient generation of the marker. In particular, ligating the oligonucleotides requires no separation or purification of the marker product. Attaching the at least one attachment oligonucleotide to the affinity reagent may comprise purification of a reaction product.

Preferably, the attachment oligonucleotide and/or the linker oligonucleotide may be attached to the affinity reagent at a specific site of the affinity reagent.

It is particularly preferred, that the step of attaching the at least one linker oligonucleotide comprises circularising unbound attachment oligonucleotides. This enables an efficient generation of the marker. Particularly, when circularising unbound attachment oligonucleotides, no separation or purification of the intermediate product (affinity reagent with attached attachment oligonucleotide) is required. In particular, the unbound attachment oligonucleotides are circularised after attaching the at least one attachment oligonucleotide to the affinity reagent. The step of circularising may comprise, for example, the addition of a circularisation oligonucleotide complementary to both ends, in particular to a sequence at a 5' and a sequence at a 3' end, of the attachment oligonucleotide. After addition of the complementary circularisation oligonucleotide, the circularised attachment oligonucleotides may preferably be ligated by addition of a ligase.

Preferably, the generated markers and the circularised unbound attachment oligonucleotides are introduced to a biological sample for analysis of the biological sample. This enables an efficient analysis of the biological sample with the generated markers. In particular, unbound affinity reagents and/or circularised attachment oligonucleotides may be washed away after introduction to the biological sample. In particular, no separation or purification of markers is necessary prior to introduction to the biological sample.

Preferably, the step of attaching the at least one linker oligonucleotide comprises removing unbound attachment oligonucleotides. In particular, the unbound attachment oligonucleotides may be removed after attaching the at least one attachment oligonucleotide to the affinity reagent. The removal of attachment oligonucleotides may be performed by chromatographic separation e.g. size exclusion, it may be based on differences in electrophoretic mobility of the unbound attachment oligonucleotide and the antibody-oligonucleotide conjugate, and/or it may be based on a selective degradation of the unbound attachment oligonucleotide, and/or it may be based on circularization of the unbound attachment oligonucleotide. Selective degradation may involve exonucleases, which attack the unbound attachment oligonucleotide from the end, which was used for conjugation to the affinity reagent. If for example the attachment oligonucleotide was conjugated with a functionalization on the 3' end, a 3'-5' exonucleases may be used to remove the unbound attachment oligonucleotide. In this case the exonucleases would have to be removed or inactivated before adding the linker oligonucleotide. Inactivation may be performed by heating for example to about 65-80° for 10-20min depending on the exonuclease that is being used. Careful evaluation of antibody tolerance to such inactivation has to be performed as some antibodies may loose biological function in these conditions.

Preferably, the at least one linker oligonucleotide comprises a first ligation sequence and the at least one attachment oligonucleotide comprises a second ligation sequence, wherein for ligating the at least one linker oligonucleotide to the at least one attachment oligonucleotide a ligase and at least one ligation oligonucleotide is added, wherein the ligation oligonucleotide comprises a sequence complementary to the first ligation sequence and the second ligation sequence. This enables an efficient ligation. In particular, the first ligation sequence and the second ligation sequence are arranged at the respective other one of either a 3' or 5' end of the respective oligos, and the ends are not attached to either affinity reagent or labelling moiety but ligated together by the ligase.

Preferably, ligating the at least one linker oligonucleotide to the at least one attachment oligonucleotide comprises adding a ligase. This enables efficiently generating the marker. In particular, the ligase may be a T4, T7, Taq ligase, or a DNA ligase I, III, IV.

Preferably, the at least one linker oligonucleotide comprises a first reaction moiety and the affinity reagent comprises a second reaction moiety, wherein the first reaction moiety and the second reaction moiety are configured to specifically react with each other. This enables efficient site-specific attachment of the linker oligonucleotide and therefore of the labelling moiety. In particular, the reaction of the reaction moieties may either be spontaneously e.g. DBCO/azide or in the presence of a catalyst e.g. Copper-dependent alkyne/azide click chemistry. In particular, the first reaction moiety and the second reaction moiety may be respective chemical reaction moieties that are configured to specifically react with each other to form a covalent bond.

Preferably, the at least one linker oligonucleotide is attached to the affinity reagent by click chemistry or NHS coupling chemistry. This enables efficient attachment of the linker oligonucleotide to the affinity reagent. Further preferred examples are maleimide and disulphide chemistries. For example, the first reaction moiety and the second reaction moiety may be click chemistry groups.

### Short Description of the Figures

Hereinafter, specific embodiments are described referring to the drawings, wherein:
- Figure 1: is a schematic view of steps to generate a marker,
- Figure 2: is a schematic view of steps to generate a marker according to a second embodiment,
- Figure 3: is a schematic view of steps to generate a marker according to a third embodiment,
- Figure 4: is a schematic view of steps to generate a marker according to a fourth embodiment,
- Figure 5: is a schematic view of steps to generate a marker according to a fifth embodiment, and
- Figure 6: is a schematic view of steps to generate a marker according to a sixth embodiment.

### Detailed Description

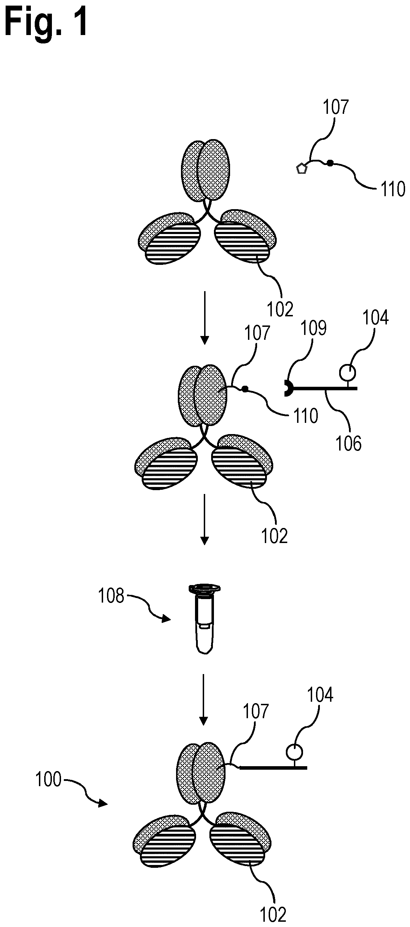

Figure 1 is a schematic view of steps of a method to generate a marker 100. The marker 100 comprises an affinity reagent 102, such as an antibody, configured to specifically bind to a target analyte (not shown) of a biological sample (not shown). The target analyte may be a protein or a nucleic acid of the biological sample, for example. The marker 100 further comprises a labelling moiety 104 and a linker oligonucleotide 106. The linker oligonucleotide 106 is attached to the affinity reagent 102 and the labelling moiety 104. Thus, the labelling moiety 104 is attached to the affinity reagent 102 via the linker oligonucleotide 106. Preferably, the attachment of the linker oligonucleotide 106 to the affinity reagent 102 and the labelling moiety 104 is via covalent bonds. This enables a robust marker 100.

The linker oligonucleotide 106 may be a single stranded nucleic acid molecule. The linker oligonucleotide 106 may comprise naturally occurring nucleic acid, such as D-DNA, and/or non-natural nucleic acid analogues, such as L-DNA or XNA.

The labelling moiety 104 may be optically detectable, in particular. Thus, the labelling moiety 104 may be a fluorophore or a polyyne for example. In a preferred embodiment, the marker 100 may comprise a plurality of the same or different labelling moieties 104. The plurality of labelling moieties 104 may all be attached to the linker oligonucleotide 106. Alternatively, the marker 100 may comprise a plurality of linker oligonucleotide 106 attached to the affinity reagent 102 and each of the labelling moieties 104 may be attached to one of the linker oligonucleotides 106 and/or each linker oligonucleotide 106 may have several labelling moieties 104 attached.

The affinity reagent 102 reagent may be a protein, such as an antibody or an antibody fragment. Alternatively, the affinity reagent 102 may be an amino acid- or nucleic acid-based aptamer. Alternatively, the affinity reagent may be a polymeric binder comprising a non-naturally occurring polymeric backbone. The affinity reagent 102 enables specifically binding the marker 100 to the target analyte of the biological sample. The marker 100, and in turn the target analyte, may then be determined and/or localised within the biological sample by detecting the labelling moiety 104 of the marker 100 in the biological sample, for example by means of a microscope in case the labelling moiety is optically detectable.

In order to generate the marker 100, parts of the marker 100, in particular the affinity reagent 102 and the linker oligonucleotide 106 comprising the attached labelling moiety 104, are attached to each other. To that end, the affinity reagent 102 may initially be activated. In particular, the affinity reagent 102 may be activated using a suitable bifunctional linker 107. Such a bifunctional linker 107 may for example comprise an NHS group, which allows coupling the linker 107 to lysine residues of the affinity reagent 102. Following the activation, the attachment oligonucleotide 106 is attached to the activated affinity reagent 102 in a reaction step 108.

Preferably, the reaction step 108 comprises forming a covalent bond between the linker oligonucleotide 106 and the affinity reagent 102. The linker oligonucleotide 106 may comprise a first reaction moiety 109 and the (activated) affinity reagent 102 may comprise a second reaction moiety 110, in particular as part of the bifunctional linker 107.

The first reaction moiety 109 and the second reaction moiety 110 may react in step 108, in particular, in the presence of a catalyst added during step 108. The catalyst may be a molecule, such as copper. Alternatively, the reaction may be driven by light, such as UV-light. In particular, the first reaction moiety and the second reaction moiety may be configured to specifically react with each other to form a covalent bond. The reaction between the linker oligonucleotide 106 and the affinity reagent 102 may be based on click chemistry or NHS coupling chemistry, as mentioned above. Further preferred examples are maleimide and disulphide chemistries. In particular, the attachment oligonucleotide 106 may comprise a functional group corresponding to the bifunctional linker 107, for example an alkyne or DBCO configured to react with the azide of the bifunctional linker 107. This enables coupling the attachment oligonucleotide 106 covalently to the affinity reagent 102, in particular via the bifunctional linker 107.

The step 108 may comprise a purification or separation of unreacted reagents from the generated marker 100 product, which may be performed by size exclusion chromatography or gel electrophoresis, for example.

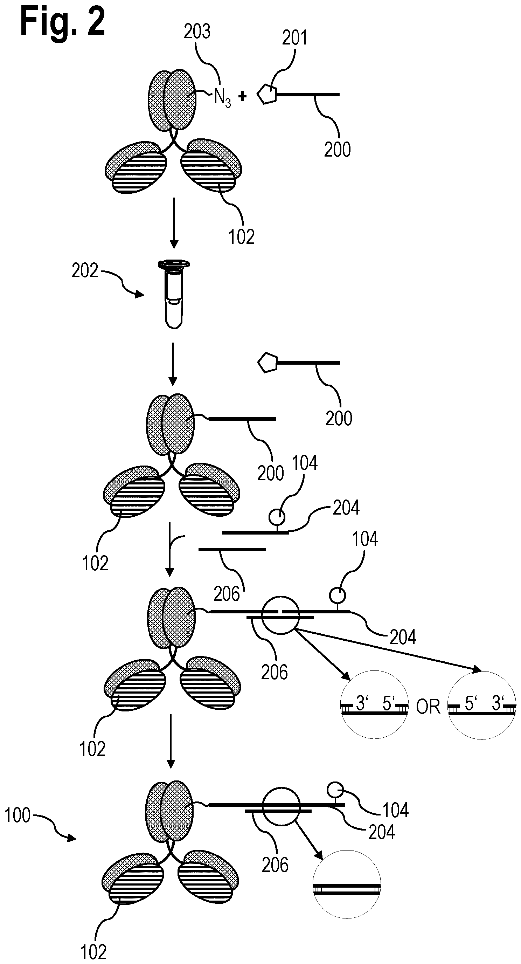

Figure 2 is a schematic view of steps of an alternative method to generate the marker 100. Initially, the affinity reagent 102 is reacted with an attachment oligonucleotide 200 in a reaction step 202. The reaction step 202 is carried out as explained for step 108 and for the linker oligonucleotide 106. Thus, the attachment oligonucleotide 200 may be attached to the affinity reagent 102 by a reaction of a first reaction moiety 201, for example an alkyne or DBCO, of the attachment oligonucleotide 200 with the second reaction moiety 203, for example an azide, of the affinity reagent 102. For example, the reaction between the attachment oligonucleotide 200 and the affinity reagent 102 may be based on click chemistry.

Subsequently, the affinity reagent 102 with the attached attachment oligonucleotide 200 is brought into contact with a linker oligonucleotide 204 comprising the labelling moiety 104 and with a ligation oligonucleotide 206. The ligation oligonucleotide 206 comprises sequences that are complementary to the attachment oligonucleotide 200 and the linker oligonucleotide 204. This enables binding the linker oligonucleotide 204 to the attachment oligonucleotide 200 via the ligation oligonucleotide 206, when the ligation oligonucleotide 206 is hybridised to the respective complementary sequences of the attachment oligonucleotide 200 and the linker oligonucleotide 204.

In a further subsequent step, the linker oligonucleotide 204 may be directly attached to the attachment oligonucleotide 200 by ligating the linker oligonucleotide 204 to the attachment oligonucleotide 200. This may comprise addition of a ligase to the linker oligonucleotide 204 and the attachment oligonucleotide 200. Further, this generates the marker 100. By means of the ligase, a covalent bond is synthesised between the linker oligonucleotide 204 and the attachment oligonucleotide 200. The ligation oligonucleotide 206 may be removed from the marker 100 subsequently.

By providing ligation oligonucleotides 206 with different nucleotide sequences, different linker oligonucleotides 204 and labelling moieties 104 may be specifically attached to the affinity reagent 102, in particular to the attachment oligonucleotide 200.

The specificity of the hybridisation and the ligation enable, in particular, generating the marker 100 without separation of unbound linker oligonucleotides 204 with labelling moieties 104.

Generally, the method according to Fig. 2 enables generating the marker 100 with a reduced loss of labelling moieties 104. In particular, the specificity of the hybridisation and ligation enable addition of stoichiometric or near stoichiometric amounts of linker oligonucleotides 204 when bringing the affinity reagent 102 with the attached attachment oligonucleotide 200 into contact with the linker oligonucleotide 204. This contrasts with the method according to Fig. 1, which generally requires a large excess of the linker oligonucleotides 106 in order to generate the marker 100.

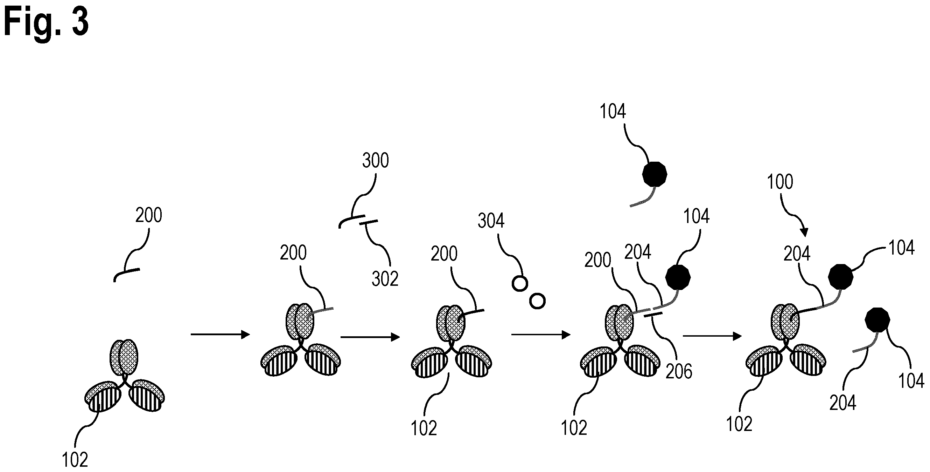

Figure 3 is a schematic view of steps of a further alternative method to generate the marker 100. Initially, the affinity reagent 102 is reacted with an attachment oligonucleotide 200 similar to the reaction step 202 described for Figure 2. However, in contrast to step 202, no separation of reactants or purification of products is carried out for the method according to Figure 3. Instead, any attachment oligonucleotides 200 that have not reacted and attached to the affinity reagent 102 are circularised. An unreacted, or unbound, attachment oligonucleotide is exemplarily shown in Fig. 3 and indicated by reference sign 300.

Subsequent to the step of attaching the attachment oligonucleotides 200 to the affinity reagent 102, a circularisation oligonucleotide 302 is brought into contact with the unbound attachment oligonucleotides 300. The circularisation oligonucleotide 302 comprises a first sequence complementary to a respective first sequence of the attachment oligonucleotides 200, 300 and a second sequence complementary to a respective second sequence of the attachment oligonucleotides 200, 300. The first sequence of the circularisation oligonucleotide 302 may be at a 5' end of the circularisation oligonucleotide 302 and the second of the circularisation oligonucleotide 302 may be at a 3' end of the circularisation oligonucleotide. The respective first sequence of the attachment oligonucleotide 300 may be at its 3' end and the respective second sequence of the attachment oligonucleotide 300 may be at its 5' end.

Subsequent to the addition of the circularisation oligonucleotide 302, a ligase may be added in order to ligate the 5' end of the unbound attachment oligonucleotide 300 to the 3' end of the unbound attachment oligonucleotide 300. This generates a circularised unbound attachment oligonucleotide 304. The removal or separation of the circularised unbound attachment oligonucleotide 304 from the affinity reagent 102 with the attachment oligonucleotide 200 is not necessary.

In a subsequent step, the linker oligonucleotide 204 with the labelling moiety 104 may be ligated to the attachment oligonucleotide 200 by means of the ligation oligonucleotide 206 and the ligase, as described for the method according to Fig. 2. Thus, the linker oligonucleotide 204 is (covalently) attached to the affinity reagent 102, in particular attached via the attachment oligonucleotide 200.

The ligation oligonucleotide 206 may preferably be configured to preferentially hybridise to the attachment oligonucleotide 200 compared to the circularisation oligonucleotide 302. In particular, this may avoid the ligation oligonucleotide 206 being prevent to hybridise to the attachment oligonucleotide 200 due to addition of excess circularisation oligonucleotides 302. The preferential hybridisation of the ligation oligonucleotide 206 may be realised by including base-pair mismatches in the complementary sequence of the circularisation oligonucleotides 302, for example. Thus, the ligation oligonucleotide 206 and the circularisation oligonucleotide 302 may have different melting temperatures, for example. Alternatively or alternatively, the ligation oligonucleotides 206 and/or the circularisation oligonucleotides 302 may comprise different natural nucleic acids and non-natural or analogous nucleic acids in order for the ligation oligonucleotide 206 to displace circularisation oligonucleotides 302 bound to the attachment oligonucleotide 200.

An alternative or additional measure may include adding the circularisation oligonucleotide 302 in stoichiometric or near stoichiometric amounts, in order to circularise all unbound attachment oligonucleotides 300.

In addition, the ligation oligonucleotide 206 may be removed from the marker 100 prior to application in a biological sample.

The method according to Fig. 3 requires no purification or separation of reactants prior to introduction of the generated marker 100 to the biological sample. Any excess reactants such as circularised unbound attachment oligonucleotides 302 or unbound linker oligonucleotides 204 with labelling moieties 104 may be removed from the biological sample by washing after the marker 100 has bound to the respective target analyte of the biological sample. This enables a particular efficient generation of the marker 100. Further, the circularised unbound attachment oligonucleotide 302 are not available for hybridisation of the linker oligonucleotides 204. This further increases the efficiency of the generation of the marker 100.

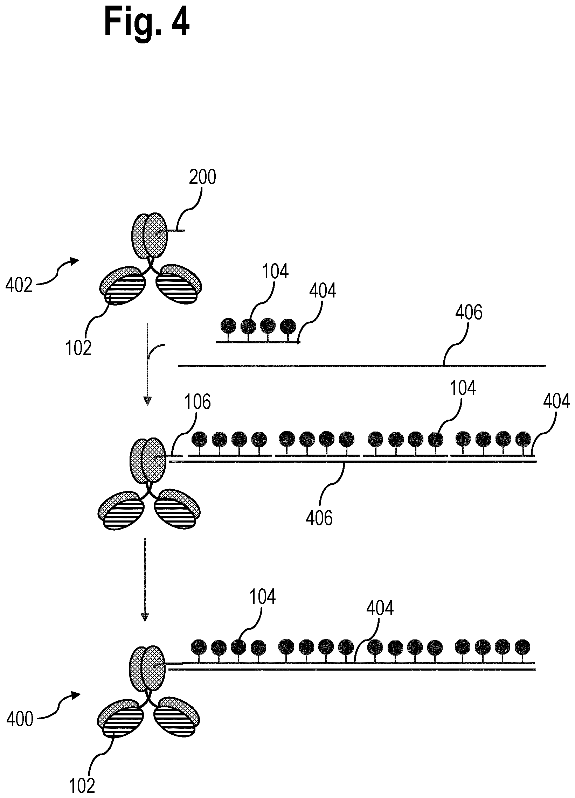

Figure 4 is a schematic view of steps of a further alternative method to generate a marker 400. Initially, the affinity reagent 102 is activated using a suitable bifunctional linker. Such a bifunctional linker may for example comprise an NHS group, which allows coupling to lysine residues, and an azide. Following to the activation, the attachment oligonucleotide 200, which comprises a corresponding functional group for example an alkyne or DBCO, is coupled covalently to the affinity reagent 102, in particular to the bifunctional linker, generating an affinity reagent oligonucleotide conjugate 402. The details of the activation step are not shown in Figure 4.

In a subsequent step a linker oligonucleotide 404 comprising a plurality of labelling moieties 104 is added to the conjugate 402 together with a ligation oligonucleotide 406. In this example shown in Figure 4, the ligation oligonucleotide 406 is a longer DNA molecule which may range from, 20 bases to 1 kbases, in particular from 20 b to 120 b, 120 b to 500 b, 500 b to 1kb in length for example, and comprises multiple sequence stretches complementary to the linker oligonucleotides 404, which are positioned in such a way along the ligation oligonucleotide 406, that their duplex formation, or hybridisation, generates a DNA double strand with multiple nicks. In order to generate the marker 400, these gaps can then be ligated in the next step forming the linker oligonucleotide 404 as a contiguous DNA strand that is on one end covalently coupled to the AR.

This embodiment allows the hybridisation of multiple linker oligonucleotides 404 on the ligation oligonucleotide 406, which is a particularly efficient way to assemble markers with high numbers of labelling moieties 104 e.g. dyes molecules.

The sequences of the linker oligonucleotides 404 may each be unique and therefore hybridise to a predetermined complementary sequence of the ligation oligonucleotide 406. This enables determining the position of the linker oligonucleotides 404 along the ligation oligonucleotide 406.

In addition, the ligation oligonucleotide 406 may be removed from the marker 400 prior to application in a biological sample.

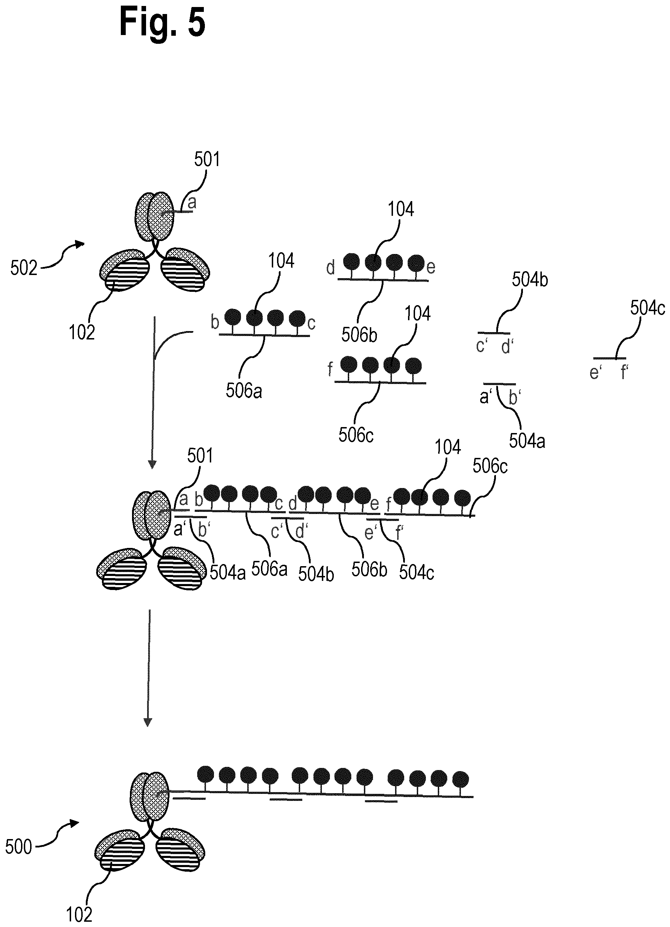

Figure 5 is a schematic view of steps of a further alternative method to generate a marker 500. Initially, an attachment oligonucleotide 501 is conjugated with the affinity reagent 102 in order to generate an affinity reagent conjugate 502. The attachment oligonucleotide 501 comprises a specific sequence a at an end opposite the end that is attached to the affinity reagent 102. The attachment oligonucleotide 501 may be attached to the affinity reagent 102 as described above. The detailed steps to attach the attachment oligonucleotide 501 to the affinity reagent 102 are not shown in Fig. 5.

Subsequently, a plurality of shorter ligation oligonucleotides 504a, 504b, 504c comprising specific sequences a' to f' at respective ends of the ligation oligonucleotides 504a, 504b, 504c are provided together with linker oligonucleotides 506a, 506b, 506c that comprise specific sequences b to f at respective ends of the linker oligonucleotides 506a, 506b, 506c. The specific sequences a' to f' of the ligation oligonucleotides 504a, 504b, 504c are complementary to the respective specific sequences a to f of the attachment oligonucleotide 501 and the linker oligonucleotides 506a, 506b, 506c. Thus, when bringing the affinity conjugate 502 into contact with the ligation oligonucleotides 504a, 504b, 504c and the linker oligonucleotides 506a, 506b, 506c, the attachment oligonucleotide 501, the ligation oligonucleotides 504a, 504b, 504c and the linker oligonucleotides 506a, 506b, 506c hybridise to each other according to their respective complementary sequences. This design allows precise control over the composition of the resulting concatenate. It may be used to attach multiple linker oligonucleotides 506a, 506b, 506c and hence labelling moieties 104.

In a subsequent step, the linker oligonucleotides 506a, 506b, 506c may be ligated together and to the attachment oligonucleotide 501 in order to generate the marker 500.

In addition, the ligation oligonucleotides 504a, 504b, 504c may be removed from the marker 500 prior to application in a biological sample.

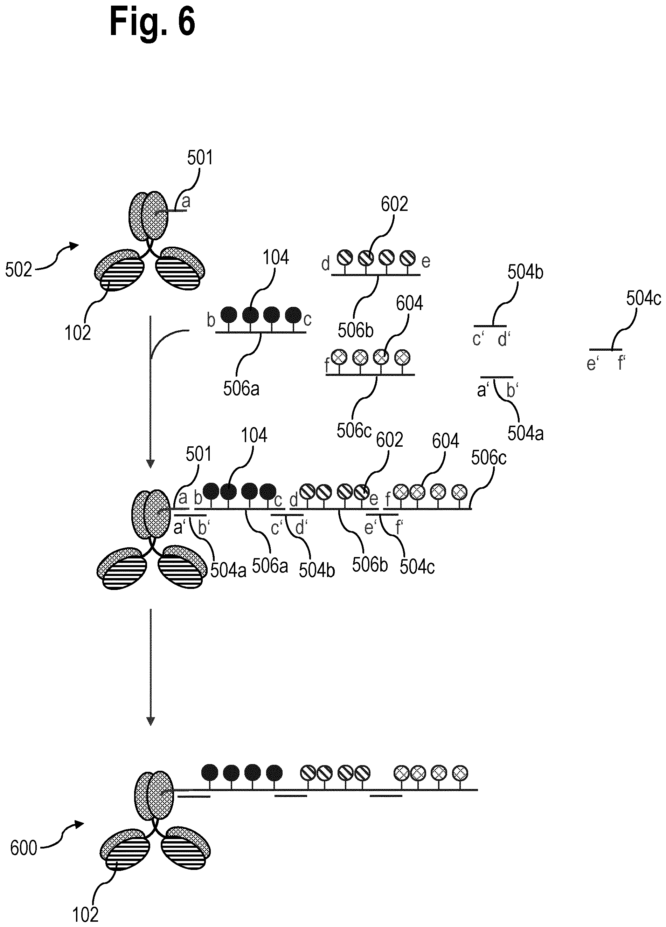

Figure 6 is a schematic view of steps of a further alternative method to generate a marker 600. In contrast to the marker 500 described with Fig. 5, the marker 600 comprises combinatorial labels. Specifically, the linker oligonucleotides 506a, 506b, 506c described above may comprise different labelling moieties 104, 602, 604. The labelling moieties 104, 602, 604 attached to each linker oligonucleotides 506a, 506b, 506c may differ from each other in at least one physical property and therefore be distinguishable from each other. For example, labelling moieties 104, 602, 604 may be fluorophores and the physical property may be an excitation or emission wavelength, or a fluorescent lifetime. In an alternative, at least one of the labelling moieties 104, 602, 604 may be polyyne or a sequence tag.

The sequences of the linker oligonucleotides 506a, 506b, 506c, in particular the sequences indicated as b to f, may each be unique and therefore hybridise to a predetermined complementary sequence of the ligation oligonucleotides 504a, 504b, 504c, in particular the sequences indicated as a' to f'. This enables determining the position of the linker oligonucleotides 506a, 506b, 506c along the ligation oligonucleotides 504a, 504b, 504c.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

100, 400, 500, 600 Marker
102 Affinity reagent
104, 602, 604 Labelling moiety
106, 204, 404, 506a, 506b, 506c Linker Oligonucleotide
107 Bifunctional linker
108, 202 Reaction step
200, 501 Attachment oligonucleotide
109, 110, 201, 203 Reaction moiety
206, 406, 504a, 504b, 504c Ligation oligonucleotide
300 Unbound attachment oligonucleotide
302 Circularisation oligonucleotide
304 Circularised unbound attachment oligonucleotide
402, 502 Affinity reagent oligonucleotide conjugate

## Claims

1. A marker (100, 400, 500, 600) for analysing a biological sample comprising:
an affinity reagent (102),
at least one labelling moiety (104, 602, 604), and
at least one linker oligonucleotide (106, 204, 404, 506a, 506b, 506c),
wherein the at least one linker oligonucleotide (106, 204, 404, 506a, 506b, 506c) is attached to the affinity reagent (102) and the at least one labelling moiety (104, 602, 604) is attached to the linker oligonucleotide (106, 204, 404, 506a, 506b, 506c).

2. The marker (100, 400, 500, 600) according to claim 1, wherein the linker oligonucleotide (106, 204, 404, 506a, 506b, 506c) is a single strand nucleic acid molecule.

3. The marker (100, 400, 500, 600) according to one of the preceding claims, comprising a plurality of labelling moieties (104, 602, 604), each one of the labelling moieties (104, 602, 604) is attached to the affinity reagent (102) by one linker oligonucleotide (106, 204, 404, 506a, 506b, 506c).

4. The marker (100, 400, 500, 600) according to one of the preceding claims, wherein a plurality of labelling moieties (104, 602, 604) is attached to the at least one linker oligonucleotide (106, 204, 404, 506a, 506b, 506c).

5. The marker (100, 400, 500, 600) according to one of the preceding claims, wherein the affinity reagent (102) is a polymer.

6. The marker (100, 400, 500, 600) according to one of the preceding claims, wherein the attachment of the linker oligonucleotide (106, 204, 404, 506a, 506b, 506c) to the affinity reagent (102) is at least one covalent bond, and the attachment of the at least one labelling moiety (104, 602, 604) to the linker oligonucleotide (106, 204, 404, 506a, 506b, 506c) is at least one covalent bond.

7. A method for generating a marker (100, 400, 500, 600) for analysing a biological sample, comprising the following steps:
providing an affinity reagent (102) and at least one linker oligonucleotide (106, 204, 404, 506a, 506b, 506c) with at least one labelling moiety (104, 602, 604) attached to the at least one linker oligonucleotide (106, 204, 404, 506a, 506b, 506c), and
attaching the at least one linker oligonucleotide (106, 204, 404, 506a, 506b, 506c) to the affinity reagent (102).

8. The method according to claim 7, wherein the step of attaching the at least one linker oligonucleotide (106, 204, 404, 506a, 506b, 506c) comprises attaching at least one attachment oligonucleotide (200, 501) to the affinity reagent (102) and further comprises ligating the at least one linker oligonucleotide (106, 204, 404, 506a, 506b, 506c) to the at least one attachment oligonucleotide (200, 501).

9. The method according to claim 8, wherein the step of attaching the at least one linker oligonucleotide (106, 204, 404, 506a, 506b, 506c) comprises circularising unbound attachment oligonucleotides (300).

10. The method according to claim 9, wherein the generated markers and the circularised unbound attachment oligonucleotides (304) are introduced to a biological sample for analysis of the biological sample.

11. The method according to one of claims 8 or 9, wherein the step of attaching the at least one linker oligonucleotide (106, 204, 404, 506a, 506b, 506c) comprises removing unbound attachment oligonucleotides (300).

12. The method according to one of the claims 8 to 11, wherein the at least one linker oligonucleotide (106, 204, 404, 506a, 506b, 506c) comprises a first ligation sequence and the at least one attachment oligonucleotide (200, 501) comprises a second ligation sequence, wherein for ligating the at least one linker oligonucleotide (106, 204, 404, 506a, 506b, 506c) to the at least one attachment oligonucleotide (200, 501) a ligase and at least one ligation oligonucleotide (206, 406, 504a, 504b, 504c) is added, wherein the ligation oligonucleotide (206, 406, 504a, 504b, 504c) comprises a sequence complementary to the first ligation sequence and the second ligation sequence.

13. The method according to one of the claims 8 to 12, wherein ligating the at least one linker oligonucleotide (106, 204, 404, 506a, 506b, 506c) to the at least one attachment oligonucleotide (200, 501) comprises adding a ligase.

14. The method according to one of the claims 7 to 13, wherein the at least one linker oligonucleotide (106, 204, 404, 506a, 506b, 506c) comprises a first reaction moiety (201) and the affinity reagent (102) comprises a second reaction moiety (203), wherein the first reaction moiety (201) and the second reaction moiety (203) are configured to react with each other.

15. The method according to one of the claims 7 to 14, wherein the at least one linker oligonucleotide (106, 204, 404, 506a, 506b, 506c) is attached to the affinity reagent (102) by click chemistry or NHS coupling chemistry.
